# EUROPÄISCHE PATENTSCHRIFT

(11) **EP 0 649 323 B1**
(45) Veröffentlichungstag und Bekanntmachung des Hinweises auf die Patenterteilung: **15.01.1997**
(21) Anmeldenummer: 93912913.6
(22) Anmeldetag: 08.06.1993
(51) Int. Cl.: A61M 25/04, A61M 5/00

(54) **PORTKATHETER**
PORT CATHETER
CATHETER IMPLANTE A RACCORD D'INJECTION

(30) Priorität: 30.06.1992 DE 4221390
(43) Veröffentlichungstag der Anmeldung: 26.04.1995
(73) Patentinhaber: Haindl, Hans, Dr., D-30974 Wennigsen (DE); LANGENFELD, Stephan, D-49090 Osnabrück (DE)
(72) Erfinder: Haindl, Hans, Dr., D-30974 Wennigsen (DE); LANGENFELD, Stephan, D-49090 Osnabrück (DE)
(74) Vertreter: Leine, Sigurd, Dipl.-Ing.
(86) Internationale Anmeldenummer: EP9301441
(87) Internationale Veröffentlichungsnummer: WO9400177

(56) Entgegenhaltungen:
- EP-A- 0 395 778
- DE-A- 3 837 779

## Beschreibung

Die Erfindung betrifft einen Portkatheter der im Oberbegriff des Anspruchs 1 genannten Art.

Durch DE 38 37 779 C2 ist ein Portkatheter der betreffenden Art bekannt, der durch eine Punktion in den Körper eines Patienten einbringbar ist, um so den Zugang zu Blutgefäßen oder anderen Körperhohlräumen zu ermöglichen, die sonst von außen nicht ohne weiteres erreichbar sind. Der Portkatheter liegt nach der Punktion unter der Haut, und seine Membran kann durch die Haut hindurch mit einer Kanüle angestochen werden. Von der Kammer in dem Portkatheter führt ein Schlauch das Medikament an den Wirkort, wo es gebraucht wird.

Ein Portkatheter der betreffenden Art ist sehr klein, um die Einbringung durch Punktierung zu ermöglichen. Um den Portkatheter trotz dieser kleinen Größe in dem Unterhaut-Fettgewebe eines Patienten zu verankern, sind bei dem Katheter der betreffenden Art an der Außenwand des Gehäuses Verankerungsschlingen vorgesehen, die nach vorn (in Einführrichtung) gerichtet durch eine Einführhülse an die Kammerwand angedruckt sind. Nach der Implantation wird die Einfuhrhülse nach der vom Einführkonus entfernten Seite abgezogen, worauf sich die während des Implantierens an die Kammerwand angelegten Verankerungsmittel aufrichten und in das benachbarte Unterhaut-Fettgewebe des Patienten eindringen. Dadurch soll eine Lagestabilisierung des Portkatheters erfolgen. Während des Abziehens der Einfüllhülse von dem Gehäuse wird letzteres mittels eines Gegenhalters in seiner bestimmungsgemäßen Lage gehalten. In dieser Schrift ist nichts darüber offenbart, wie die Verankerungsschlingen ausgebildet, an dem Gehäuse angebracht sind und wie erreicht wird, daß sie sich bei Zurückziehen der Einführhülse aufrichten, um so in das benachbarte Unterhaut-Fettgewebe des Patienten einzudringen.

Der Erfindung liegt die Aufgabe zugrunde, einen Portkatheter der betreffenden Art zu schaffen, bei dem die Verankerungsschlingen einfach ausgebildet, zu fertigen und an dem Gehäuse sicher anzubringen sind und bei denen gewahrleistet ist, daß sie sich im implantierten Zustand bei Zurückziehen der Einführungshülse sicher entfalten und ein hohes Maß an Verankerungssicherheit gewährleisten.

Die der Erfindung zugrundeliegende Aufgabe wird durch die im Kennzeichen des Anspruchs 1 angegebene Lehre gelöst.

Grundgedanke dieser Lehre ist es, die Verankerungsschlingen aus Federdraht herzustellen, wobei die Enden der Verankerungsschlingen Jeweils das Gehäuse an zwei axial voneinander entfernten Stellen umschlingen. Dadurch ist ein hohes Maß an Biegesteifigkeit quer zur Längsachse des Portkatheters gegeben. In Umfangsrichtung lassen sich die Verankerungsschlingen praktisch in Weiterführung ihrer Umschlingung um das Gehäuse herumbiegen. Mit einer hohen Biegesteifigkeit im Sinne einer Verankerung gegen Bewegungen in Achsrichtung verbindet sich daher eine gute Biegsamkeit in Umfangsrichtung, in der eine besondere Verankerungssicherheit nicht erforderlich ist.

Die Mittel zum Zusammenhalten der Verankerungsschlingen können in verschiedener Weise ausgebildet sein. Eine zweckmäßige Ausführungsform besteht darin, daß eine Gabel mit zwei Zinken vorgesehen ist, die die Enden der zusammengefalteten Schlingen zusammenhalten, wobei die Gabel einen Stiel aufweist, der sich über das dem Konus abgewandte Ende des Gehäuses hinaus erstreckt. Die Enden der Zinken sind dabei zweckmäßigerweise etwas aufeinander zu gebogen. Nach der Implantation wird das hintere Ende des Stiels der Gabel ergriffen und abgezogen, so daß die Zinken die Verankerungsschlingen freigeben, die sich so entfalten können.

Eine andere Ausbildungsform der Mittel zum Zusammenhalten der Verankerungsschlingen besteht in einem Faden, dessen eines Ende die Enden der beiden zusammengefalteten Verankerungsschlingen umschlingt und mit einer Schleife zusammengehalten ist, die mittels des anderen, sich über das dem Konus abgewandte Ende des Gehäuses 1 hinaus erstreckende Ende des Fadens lösbar ist. Diese Ausführungsform ist besonders einfach und preiswert.

Eine andere Möglichkeit der Verwendung eines Fadens besteht gemäß einer anderen Weiterbildung der Erfindung darin, daß ein Ende des Fadens an einer der Verankerungsschlingen befestigt ist, während das andere Ende des Fadens durch eine Öse an dem Ende der anderen Verankerungsschlinge und dann über das dem Konus abgewandte Ende des Gehäuses hinaus geführt ist. Durch Zug an dem Faden können die Verankerungsschlingen in zusammengefaltetem Zustand gehalten werden, wenn der Portkatheter implantiert wird. Wird danach der Faden vom Zug entlastet, so werden die Verankerungsschlingen freigegeben, die sich so entfalten und den Portkatheter wirksam verankern können. Diese Ausführungsform mit einem Faden hat den großen Vorteil, daß mit seiner Hilfe auch das Wiederentfernen des Portkatheters erleichtert ist. Hierzu wird das freie Ende des Fadens nach Punktieren aufgesucht, herausgezogen und unter Zug gesetzt. Durch diesen Zug werden die Verankerungsschlingen wieder zusammengefaltet, so daß ein einfaches Entfernen möglich ist. Besonders vorteilhaft ist es, wenn der Faden die Enden der beiden Verankerungsschlingen mehrmals umschlingt, bevor er aus der Öse nach hinten geführt ist. Dadurch tritt eine Flaschenzugwirkung auf, so daß bei geringen Zugkräften an dem Faden bereits große Kräfte zum Zusammenhalten oder Zusammenführen der Verankerungsschlingen erzeugt werden können.

Bei einer anderen Weiterbildung der Erfindung bestehen die Mittel zum Zusammenhalten der Verankerungsschlingen im zusammengefalteten Zustand in einer Axialnut, in die die Enden der Verankerungsschlingen in dem zusammengebogenen Zustand eingerastet sind. Zum Ausrasten dient ein Faden, der an einer der Schlingen befestigt ist, mit denen die Verankerungsschlingen an dem Gehäuse 1 gehalten sind. Durch Zug an dem Faden kommen die Verankerungsschlingen frei und können sich daher entfalten.

Eine weitere zweckmäßige Ausführungsform der Erfindung besteht darin, daß die Verankerungsschlingen im zusammengefalteten Zustand durch einen sie umgebenden zylindrischen Ring zusammengehalten sind, der aus einem Material besteht, das sich im Körper schnell auflöst, so daß dadurch die Verankerungsschlingen freigegeben werden.

Als Mittel zum Zusammenhalten der Verankerungsschlingen kann auch ein Schlauch dienen, der die zusammengefalteten Verankerungsschlingen umschließt und ausreichend nach hinten vorsteht, so daß er nach der Implantation abgezogen werden kann, so daß dadurch die Verankerungsschlingen freikommen und sich entfalten. Der Schlauch kann auch so ausgebildet sein, daß er sich in Längsrichtung teilen läßt, was eine Lösung von dem Gehäuse 1 begünstigt.

Schließlich können die Mittel zum Zusammenhalten der Verankerungsschlingen durch eine an sich bekannte Einführhülse gebildet sein, die über das Gehäuse bis über die zusammengefalteten Verankerungsschlingen schiebbar ist und über das dem Konus abgewandte Ende des Gehäuses vorsteht. Diese Einfuhrhülse erleichtert die Manipulation bei der Implantation und ermöglicht darüberhinaus weitere zweckmäsige Ausführungsformen dieser Weiterbildung.

Eine weitere zweckmäßige Ausführungsform dieser Weiterbildung besteht darin, daß die beiden Verankerungsschlingen die Schenkel eines V's bilden, das in Richtung auf den Konus offen ist. Dies hat den Vorteil, daß beim Zurückziehen der Einführhülse sich die Verankerungsschlingen langsam öffnen und nicht plötzlich auseinanderschnappen, was eine Traumatisierung des umgebenden Gewebes zur Folge haben kann. Dies gilt auch bei Verwendung eines Schlauches zum Zusammenhalten der Verankerungsschlingen. Besonders zweckmäßig ist dabei eine Ausführungsform, bei der die Einführhülse mit einem elastischen Teil reibschlüssig auf dem Gehäuse aufliegt. Dies verhindert ein plötzliches selbständiges Rückschnellen der Einführhülse, wenn dies nicht durch den behandelnden Arzt kontrolliert wird, und damit auch ein immer noch schnelles Auseinanderbewegen der Verankerungsschlingen. Der Reibschluß läßt sich zweckmäßigerweise durch eine Zunge bewirken, die durch Einschnitte in der Hülse gebildet ist und reibschlüssig auf der Außenwandung des Gehäuses oder eines Teils davon aufliegt.

Die Ausbildung der Zunge ermöglicht eine zweckmäßige Weiterbildung, bei der die Membran durch einen nach innen gerichteten Kragen einer Hülse gegen das hintere Ende des Gehäuses gepreßt ist, während die Hülse nach innen gerichtete, widerhakenformige Eindrückungen aufweist, die in im wesentlichen komplimentäre Vertiefungen in der Außenwandung des Gehäuses eingreifen. Durch die Hülse ist zunächst eine sichere und dichte Halterung der Membran gewährleistet. Gleichzeitig ermöglicht sie aber eine Weiterbildung, bei der die Zunge eine nach innen gerichtete Kröpfung aufweist, die in eine widerhakenförmige Vertiefung in der Hülse eingreift und so die Einführhülse in Drehrichtung formschlüssig mit dem Gehäuse verbindet. Diese drehsichere Verbindung begünstigt zunächst das Einführen des erfindungsgemäßen Portkatheters. Gleichzeitig ist sie aber zweckmäßig, wenn der Konus als Überwurfmutter zur Befestigung eines Schlauchkatheters ausgebildet ist. Bei Anbringung des Schlauchkatheters läßt sich daher der Portkatheter mittels der Einführhülse gegen die Aufschraubkraft der Überwurfmutter sicher halten. Die Überwurfmutter hat zweckmäßigerweise einen Durchmesser, der gleich oder etwas größer ist als der Durchmesser der Einführhülse. Dadurch ist sichergestellt, daß beim Einführen des Portkatheters keine einen Widerstand bildende Kante vorhanden ist.

Anhand der Zeichnung soll die Erfindung an Ausführungsbeispielen näher erläutert werden.
- Fig. 1: zeigt einen Portkatheter der betreffenden Art in Seitenansicht,
- Fig. 2: zeigt den Portkatheter gemäß Fig. 1 in gleicher, aber vergrößerter Darstellung mit teilweise eingeschobener Einführhülse,
- Fig. 3: ist ein Längsschnitt durch Fig. 2, jedoch mit vollständig aufgeschobener Einführhülse, und
- Fig. 4: zeigt die Verankerungsschlingen allein in axialer Darstellung,
- Fig. 5: zeigt in Seitenansicht ein zweites Ausführungsbeispiel der Erfindung mit einer Gabel zum Zusammenhalten der gebogenen Verankerungsschlingen,
- Fig. 6: zeigt in ähnlicher Darstellung wie Fig. 5 ein drittes Ausführungsbeispiel der Erfindung mit einem Faden zum Zusammenhalten der gebogenen Verankerungsschlingen,
- Fig. 7: zeigt in ähnlicher Darstellung wie Fig. 5 ein viertes Ausführungsbeispiel der Erfindung mit einem Faden zum Zusammenhalten der Verankerungsschlingen,
- Fig. 8: zeigt das Ausführungsbeispiel gemäß Fig. 7 mit auseinandergefalteten Verankerungsschlingen,
- Fig. 9: zeigt ein fünftes Ausführungsbeispiel der Erfindung mit einer Axialnut zum Zusammenhalten der Schlingen,
- Fig. 10: zeigt ein sechstes Ausführungsbeispiel der Erfindung,
- Fig. 11: zeigt ein siebtes Ausführungsbeispiel der Erfindung und
- Fig. 12: zeigt ein achtes Ausführungsbeispiel der Erfindung.

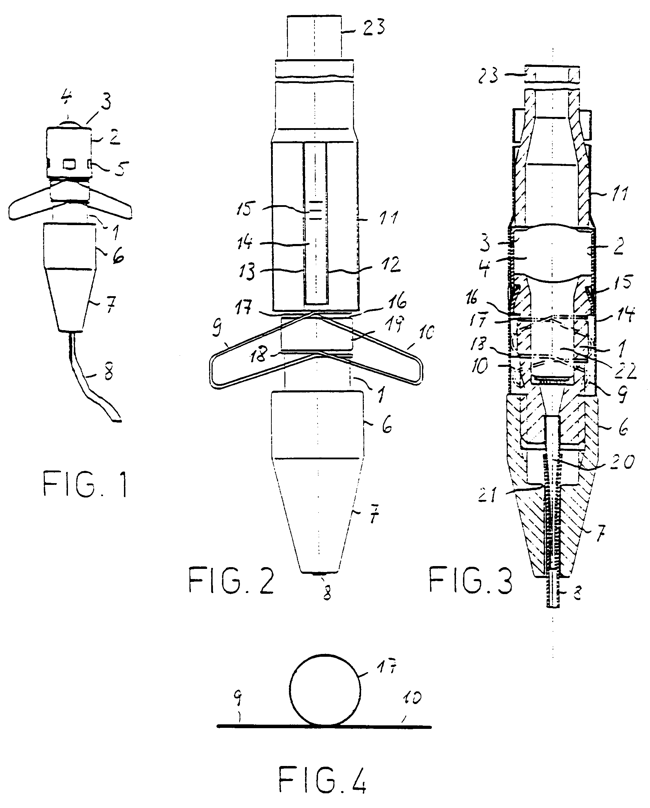

Fig. 1 zeigt ein praktisches Ausführungsbeispiel eines Portkatheters in etwa 1,5facher Vergrößerung. Ein zylindrisches Gehäuse 1 weist an seinem einen Ende eine Kappe 2 mit einem nach innen gerichteten Kragen 3 auf, der eine Membran 4 aus Silikonkautschuk gegen das in dieser Zeichnung nicht dargestellte hintere Ende des Gehäuses 1 dicht andrückt. Die Kappe 2 ist durch widerhakenförmige Eindrückungen 5 an dem Gehäuse 1 gehalten. An dem der Membran 4 abgewandten Ende des Gehäuses 1 ist auf dieses eine Überwurfmutter 6 mit einem Konus 7 zur Erleichterung einer Implantation durch Punktion vorgesehen. Die Überwurfmutter 3 dient zum Festschrauben eines Schlauchkatheters 8. An dem Gehäuse 1 befinden sich zwischen der Kappe 2 und der Überwurfmutter 6 zwei Verankerungsschlingen 9 und 10.

Fig. 2 zeigt den Portkatheter gemäß Fig. 1 in vergrösserter Darstellung, wobei jedoch auf das Ende des Gehäuses 1 mit der Kappe 2 eine Einführhülse 11 aufgeschoben ist. Die Einführhülse 11 weist Einschnitte 12 und 13 auf, so daß in ihrer Wandung eine Zunge 14 gebildet ist, die eine Kröpfung 15 aufweist, die reibschlüssig auf der Kappe 2 aufliegt.

Des weiteren ist aus Fig. 2 zu ersehen, daß die Verankerungsschlingen 9 und 10 im Bereich einer Nut 16 in dem Gehäuse 1 dieses mit einer Schlinge 17 umschlingen und so an einer ersten Stelle sowohl gegen axiale als auch gegen radiale Bewegungen gesichert sind. Mit einer zweiten Schlinge 18 umschlingen die Verankerungsschlingen 9 und 10 das Gehäuse 1 an einer zweiten, von der ersten Stelle entfernten Stelle, wo sie in gleicher Weise gegen verschiedene Bewegungen gesichert sind. Zwischen Verankerungsschlingen 17 und 18 erstreckt sich ein im Durchmesser verdickter Teil 19 des Gehäuses 1. Trotz der sicheren Verankerung der Verankerungsschlingen 9 und 10 an dem Gehäuse 1 an zwei axial voneinander entfernten Stellen lassen sich die Verankerungsschlingen 9 und 10 in Weiterführung der Verankerungsschlingen 17 und 18 um das Gehäuse 1 herumbiegen, ohne daß sie den elastischen Verformungsbereich verlassen. In diesem Zustand läßt sich die Einführhülse 11 weiter in Richtung auf die Überwurfmutter 6 über die Verankerungsschlingen 9 und 10 hinwegschieben. Dieser Zustand ist in Fig. 3 gezeigt.

Aus Fig. 3 ist zu ersehen, daß die Verankerungsschlingen 9 und 10 im gebogenen Zustand innerhalb der Einführhülse 11 gefangen sind. Gleichzeitig ist zu ersehen, daß die Kröpfung 15 der Zunge 14 in eine Eindrückung 5 der Hülse 2 eingreift, so daß dadurch die Einführhülse 11 drehsicher mit dem Gehäuse 1 verbunden ist. Dieses ist vorteilhaft für das Aufschrauben der Überwurfmutter 6 zur Befestigung des Schlauchkatheters 8, wenn dieser auf einen trompetenförmigen Stutzen 20 aufgeschoben und auf diesem mittels einer Quetschkante 21 festgeklemmt werden soll, um somit den Innenraum des Katheters 8 mit einer Kammer 22 in dem Gehäuse 1 zu verbinden, in die eine Injektionsflüssigkeit eingespritzt wird, nachdem eine Injektionsnadel durch die Membran 4 gestochen worden ist.

Wie aus den Fig. 2 und 3 zu ersehen ist, ist das hintere Ende der Einführhülse 11 verkürzt dargestellt. Im Inneren der Einfuhrhülse 11 ist eine kleine Schubstange 23 gezeigt, mit deren Hilfe eine Gegenkraft auf das Gehäuse 1 ausgeübt werden kann, wenn die Einführhülse 11 nach der Implantation zurückgezogen wird.

Fig. 4 zeigt in Einzeldarstellung die Verankerungsschlingen 9 und 10 in der in Fig. 2 gezeigten Spreizlage in Axialdarstellung mit Blick auf die Schlinge 17, hinter der - nicht sichtbar - die Schlinge 18 liegt. Verankerungsschlingen 9, 10 und Verankerungsschlingen 17, 18 bestehen zweckmäßigerweise aus einem endlosen Stück.

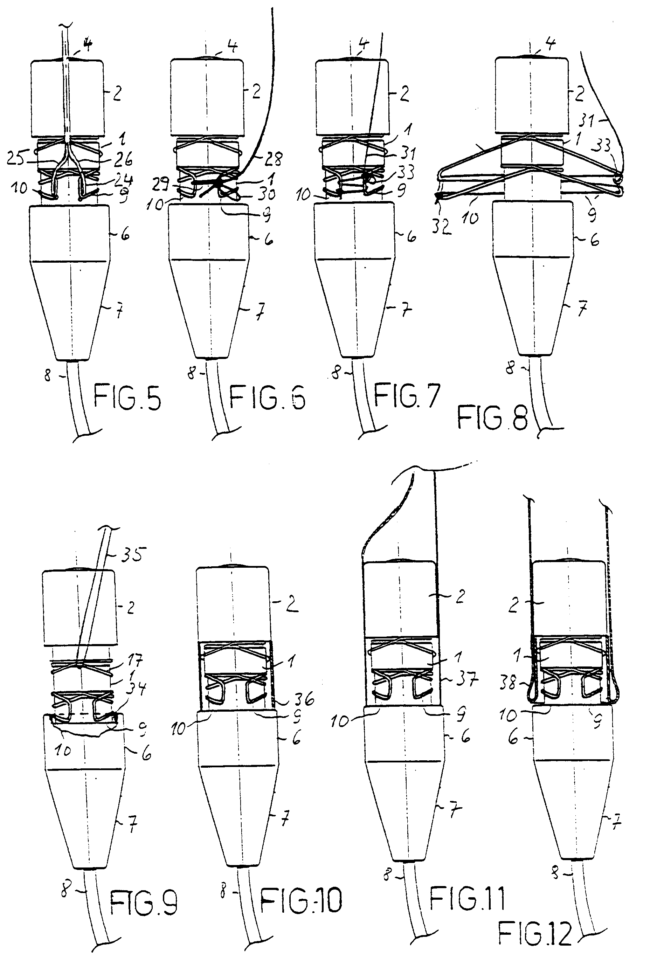

In den Fig. 5 bis 12 sind verschiedene Ausführungsbeispiele der Erfindung in Seitenansicht ähnlich gemäß Fig. 1 gezeigt mit unterschiedlichen Mitteln zum Zusammenhalten der zusammengefalteten Verankerungsschlingen. Gleiche Teile sind mit gleichen Bezugsziffern versehen wie in Fig. 1 bis 3.

Bei dem Ausführungsbeispiel gemäß Fig. 5 sind die zum Zwecke der Implantation zusammengefalteten und um das Gehause 1 gebogenen Verankerungsschlingen 9 und 10 durch Zinken 24 und 25 einer Gabel 26 zusammengehalten, deren Stiel 27 sich über das dem Konus 7 abgewandte Ende hinaus erstreckt, so daß die Gabel mittels des Stieles 27 nach der Implantation ab- und herausgezogen werden kann, so daß die Schlingen 9 und 10 freigegeben werden und sich entfalten können. Die Zinken 24 und 25 sind an ihren Enden geringfügig nach innen gebogen, so daß die Zinken bei der Implantation nicht herausrutschen können.

Bei dem Ausführungsbeispiel gemäß Fig. 6 sind die Verankerungsschlingen 9 und 10 in zusammengefaltetem Zustand durch einen Faden 28 zusammengehalten, dessen eines Ende 29 die Enden der Verankerungsschlingen 9 und 10 umschließt und mittels einer Schleife 30 zusammengehalten ist, die mittels des Fadens 28 nach der Implantation lösbar ist, so daß die Verankerungsschlingen 9 und 10 freigegeben werden. Der Faden 28 erstreckt sich über das dem Konus 7 abgewandte Ende des Gehauses 1 hinaus, so daß der Faden nach der Implantation noch außerhalb des Körpers liegt und ergriffen werden kann.

Bei dem Ausführungsbeispiel gemäß Fig. 7 werden die zusammengefalteten Verankerungsschlingen 9 und 10 durch einen Faden 31 zusammengehalten, der an einem Punkt 32 an der Verankerungsschlinge 10 befestigt ist und mehrmals zwischen den Verankerungsschlingen 9 und 10 umherläuft, bis er durch eine in der Verankerungsschlinge 9 ausgebildete Öse 33 nach hinten über das hintere Ende des Gehäuses 7 hinausgeführt ist. Diese Ausführungsform hat den Vorteil, daß die Enden der zusammengefalteten Verankerungsschlingen 9 und 10 flaschenzugartig zusammengehalten sind. Da der Faden 31 nach der Implantation in ausreichender Länge im Körper verbleiben kann, ist es möglich, zum Zwecke der Entfernung des Portkatheters nach erneuter Punktierung das Ende des Fadens 31 aufzusuchen, aus dem Körper herauszuziehen und Zug darauf auszuüben, wodurch die Verankerungsschlingen 9 und 10 wieder zusammengefaltet und um das Gehäuse 1 gebogen werden können. Der auseinandergefaltete Zustand der Verankerungsschlingen 9 und 10 ist in Fig. 8 gezeigt, wo auch besonders deutlich die Öse 33 erkennbar ist.

Fig. 9 zeigt ein Ausführungsbeispiel, bei dem die Enden der zusammengefalteten Verankerungsschlingen 9 und 10 in einer Axialnut 34 zwischen der Überwurfmutter 6 und dem Gehäuse 1 eingeschoben sind, so daß sie gegen Auseinanderfalten gesichert sind. Um die Schlinge 17 ist ein Faden 35 gezogen, mit dessen Hilfe die Schlinge 17 und damit auch die Verankerungsschlingen 9 und 10 nach hinten ziehbar sind, so daß sie aus der Axialnut 34 heraustreten und sich entfalten können.

Bei der Ausführungsform gemäß Fig. 10 sind die Verankerungsschlingen 9 und 10 im zusammengebogenen Zustand durch einen zylindrischen Ring 36 gehalten, der aus einem Material besteht, das sich nach der Implantation im Körper auflöst, so daß die Verankerungsschlingen 9 und 10 freigegeben werden und sich entfalten können.

Fig. 11 zeigt eine Ausführungsform, bei der die Verankerungsschlingen 9 und 10 im zusammengefalteten Zustand durch einen Schlauch 37 gehalten sind, der sich über das hintere Ende des Portkatheters hinaus erstreckt und so lang bemessen ist, daß er nach der Implantation ergriffen und der Schlauch 37 somit von den Verankerungsschlingen 9 und 10 abgezogen werden kann, so daß sich diese entfalten können.

Die Ausführungsform gemäß Fig. 12 entspricht weitgehend der gemäß Fig. 11 mit dem Unterschied, daß ein Schlauch 38 verwendet ist, der im Bereich der Verankerungsschlingen 9 und 10 nach innen umgekrempelt ist und außerdem aus einem Material besteht, das in Längsrichtung aufreißbar ist.

## Patentansprüche

1. Portkatheter, mit einem im wesentlichen zylindrischen Gehäuse (1), in dem sich eine Kammer (22) befindet, die auf einer Seite durch eine mit einer Injektionsnadel durchstechbare Membran (4) verschlossen und auf der anderen Seite über Verbindungsmittel mit einem Schlauchkatheter (8) verbindbar ist, mit einem Konus (7) an dem der Membran (4) abgewandten Ende des Gehäuses (1) zur Implantation des Katheters durch Punktion, mit zwei Verankerungsschlingen (9, 10), die von der Außenwand des Gehäuses (1) vorstehen und zusammenfaltbar sind, und mit Mitteln zum Halten der Verankerungsschlingen (9, 10) im zusammengefalteten Zustand, **dadurch gekennzeichnet**, daß die Verankerungsschlingen (9, 10) durch einen Federdraht gebildet sind, der das Gehäuse (1) an einer ersten Stelle umschlingt, sich von dieser aus von dem Gehäuse (1) an zwei sich gegenüberliegenden Seiten weg erstreckend in Form von zwei Verankerungsschlingen (9, 10) zu einer zweiten Stelle, die axial entfernt von der ersten Stelle ist, zu dem Gehäuse (1) zurückläuft und dort das Gehäuse (1) nochmals umschlingt.

2. Portkatheter nach Anspruch 1, **dadurch gekennzeichnet**, daß die beiden Verankerungsschlingen (9, 10) zum Zwecke des Zusammenfaltens in Umschlingungsrichtung um das Gehäuse (1) biegbar sind.

3. Portkatheter nach Anspruch 1, **dadurch gekennzeichnet**, daß die Mittel zum Zusammenhalten der Verankerungsschlingen (9, 10) durch eine Gabel (26) mit zwei Zinken (24, 25) gebildet sind, die die Enden der zusammengefalteten Verankerungsschlingen (9, 10) zusammenhalten, und daß die Gabel (26) einen Stiel (27) oder einen an der Gabel befestigten Faden aufweist, der sich über das dem Konus (7) abgewandte Ende des Gehäuses (1) hinaus erstreckt.

4. Portkatheter nach Anspruch 3, **dadurch gekennzeichnet**, daß die Enden der Zinken (24, 25) etwas aufeinander zu gebogen sind.

5. Portkatheter nach Anspruch 1, **dadurch gekennzeichnet**, daß die Mittel zum Zusammenhalten der Verankerungsschlingen (9, 10) durch einen Faden (28) gebildet sind, dessen eines Ende die Enden der beiden zusammengefalteten Verankerungsschlingen (9, 10) umschlingt und mit einer Schleife (30) zusammengehalten ist, die mittels des anderen, sich über das dem Konus (7) abgewandte Ende des Gehäuses (1) hinaus erstreckende Ende des Fadens (28) lösbar ist.

6. Portkatheter nach Anspruch 1, **dadurch gekennzeichnet**, daß die Mittel zum Zusammenhalten der Verankerungsschlingen durch einen Faden (31) gebildet sind, dessen eines Ende an einer der Verankerungsschlingen (10) befestigt ist, und daß das andere Ende des Fadens (31) durch eine Öse (33) an dem Ende der anderen Verankerungsschlinge (9) und dann über das dem Konus (7) abgewandte Ende des Gehäuses (1) hinaus geführt ist.

7. Portkatheter nach Anspruch 1, **dadurch gekennzeichnet**, daß die Mittel zum Zusammenhalten der Verankerungsschlingen (9, 10) durch eine Axialnut (34) gebildet sind, in die die Enden der Verankerungsschlingen (9, 10) in dem zusammengebogenen Zustand eingerastet sind, und daß an den Schlingen (17, 18), insbesondere im Umschlingungsbereich des Gehäuses (1), ein Faden (35) befestigt ist, der sich über das dem Konus (7) abgewandte Ende des Gehäuses (1) hinaus erstreckt und mit dessen Hilfe die Verankerungsschlingen (9, 10) aus der Axialnut (34) herausziehbar sind.

8. Portkatheter nach Anspruch 1, **dadurch gekennzeichnet**, daß die Mittel zum Zusammenhalten der Verankerungsschlingen (9, 10) durch einen zylindrischen Ring (36) gebildet sind, der die zusammengefalteten Verankerungsschlingen (9, 10) umschließt und aus einem Material besteht, das sich nach Implantation des Portkatheters auflöst.

9. Portkathether nach Anspruch 1, **dadurch gekennzeichnet**, daß die Mittel zum Zusammenhalten der Verankerungsschlingen (9, 10) durch einen Schlauch (37, 38) gebildet sind, der die zusammengefalteten Verankerungsschlingen (9, 10) umschließt, das Gehäuse (1) zu dem dem Konus (7) abgewandten Ende hin überragt und von diesem abziehbar ist.

10. Portkatheter nach Anspruch 9, **dadurch gekennzeichnet**, daß der Schlauch (38) aus einem in Längsrichtung aufreißbaren Material besteht.

11. Portkatheter nach Anspruch 9, **dadurch gekennzeichnet**, daß das die zusammengefalteten Verankerungsschlingen (9, 10) umschließende Ende des Schlauches (38) im Bereich der Verankerungsschlingen (9, 10) nach innen umgekrempelt ist.

12. Portkatheter nach Anspruch 1, **dadurch gekennzeichnet**, daß die Mittel zum Zusammenhalten der Verankerungsschlingen (9, 10) durch eine an sich bekannte Einführhülse (11) gebildet sind, die über das Gehäuse (1) bis über die zusammengefalteten Verankerungsschlingen (9, 10) schiebbar ist und über das dem Konus (7) abgewandte Ende des Gehäuses (1) vorsteht.

13. Portkatheter nach Anspruch 12**, dadurch gekennzeichnet**, daß die beiden Verankerungsschlingen (9, 10) die Schenkel eines V's bilden, das in Richtung auf den Konus (7) offen ist.

14. Portkatheter nach Anspruch 12 und 13, **dadurch gekennzeichnet**, daß die Einführhülse (11) mit einem elastischen Teil reibschlüssig auf dem Gehäuse (1) aufliegt.

15. Portkatheter nach Anspruch 14, **dadurch gekennzeichnet**, daß das elastische Teil wenigstens eine Zunge (14) ist, die durch Einschnitte (12, 13) in der Einführhülse (11) gebildet ist.

16. Portkatheter nach Anspruch 15, **dadurch gekennzeichnet**, daß die Membran (4) durch einen nach innen gerichteten Kragen (3) einer vorzugsweise aus Metall bestehenden Hülse (2) gegen das hintere Ende des Gehäuses (1) gepreßt ist, wobei die Einführhülse (11) nach innen gerichtete, widerhakenförmige Eindrückungen (5) aufweist, die im wesentlichen komplementäre Vertiefungen in der Außenwandung des Gehäuses (1) hintergreifen.

17. Portkatheter nach Anspruch 16, **dadurch gekennzeichnet**, daß die Zunge (14) eine nach innen gerichtete Kröpfung (15) aufweist, die in die widerhakenförmige Vertiefung (5) in der Einführhülse (11) eingreift und so die Einführhülse (11) in Drehrichtung formschlüssig mit dem Gehäuse (1) verbindet.

18. Portkatheter nach Anspruch 17, **dadurch gekennzeichnet**, daß der Konus (7) an einer Überwurfmutter (6) zur Befestigung eines Schlauchkatheters (8) ausgebildet ist.

19. Portkatheter nach Anspruch 17, **dadurch gekennzeichnet**, daß die Überwurfmutter (6) einen Durchmesser hat, der gleich oder etwas größer ist als der Durchmesser der Einführhülse (11).

## Claims

1. Port catheter comprising a substantially cylindrical housing (1), in which is situated a chamber (22) closed at one side by a membrane (4), which can be pierced by an injection needle, and connectable at the other side through connecting means to a tube catheter (8), a cone (7) at the end of the housing (1) facing away from the membrane (4) for the implantation of the catheter through a puncture, two anchoring loops (9, 10), which project from the outer wall of the housing (1) and may be folded, and means for holding the anchoring loops (9, 10) in the folded state, characterised in that the anchoring loops (9, 10) are formed by a spring wire which is coiled round the housing (1) at a first location, extends from it, away from the housing (1), on two mutually opposite sides in the form of two anchoring loops (9, 10) and returns back to the housing (1) at a second location, which is axially spaced from the first location, and is there coiled again round the housing (1).

2. Port catheter according to claim 1, characterised in that, to make the two anchoring loops (9, 10) foldable, they are bendable round the housing (1) in the direction of coiling round.

3. Port catheter according to claim 1, characterised in that the means for holding the anchoring loops (9, 10) are formed by a fork (26) having two prongs (24, 25) which hold together the ends of the folded anchoring loops (9, 10), and that the fork (26) has a handle (27) or a filament fixed to the fork, which extends out beyond the end of the housing (1) facing away from the cone (7).

4. Port catheter according to claim 3, characterised in that the ends of the prongs (24, 25) are somewhat bent towards each other.

5. Port catheter according to claim 1, characterised in that the means for holding the anchoring loops (9, 10) are formed by a filament (28) on end of which loops over the ends of the two folded anchoring loops (9, 10) and is held together by a bowknot (30), which may be released by the other end of the filament (28) which extends out beyond the end of the housing (1) facing away from the cone (7).

6. Port catheter according to claim 1, characterised in that the means for holding the anchoring loops are formed by a filament (31) one end of which is fixed to one of the anchoring loops (10) and that the other end of the filament (31) is passed thorough an eye (33) at the end of the other anchoring loop (9) and then passes out beyond the end of the housing (1) facing away from the cone (7).

7. Port catheter according to claim 1, characterised in that the means for holding the anchoring loops (9, 10) are formed by an axial groove (34) in which are engaged, in the folded state, the ends of the anchoring loops (9, 10), and that a filament (35) is attached to the coils (17, 18), particularly in the region where they are coiled round the housing (1), the filament (35) extending out beyond the end of the housing (1) facing away from the cone (7) and by means of the filament the anchoring loops (9, 10) may be pulled out of the axial groove (34).

8. Port catheter according to claim 1, characterised in that the means for holding the anchoring loops (9, 10) are formed by a cylindrical ring (36) which surrounds the folded anchoring loops (9, 10) and is made of a material which is absorbed after implantation of the port catheter.

9. Port catheter according to claim 1, characterised in that the means for holding the anchoring loops (9, 10) are formed by a tube (37, 38) which surrounds the folded anchoring loops (9, 10), extends beyond the end of the housing (1) facing away from the cone (7) and may be pulled off therefrom.

10. Port catheter according to claim 9, characterised in that the tube (38) is made of a material which may be torn in longitudinal direction.

11. Port catheter according to claim 9, characterised in that the end of the tube (38) surrounding the folded anchoring loops (9, 10) is in the region of the anchoring loops (9, 10) inwardly folded back.

12. Port catheter according to claim 1, characterised in that the means for holding the anchoring loops (9, 10) are formed by an insertion sleeve (11) known per se which can be slid over the housing (1) beyond the folded anchoring loops (9, 10) and projects beyond the end of the housing (1) facing away from the cone (7).

13. Port catheter according to claim 12, characterised in that the two anchoring loops (9, 10) form the arms of a V which is open in the direction to the cone (7).

14. Port catheter according to claim 12 and 13, characterised in that the insertion sleeve (11) is positioned on the housing (1) with an elastic portion and held there by frictional force.

15. Port catheter according to claim 14, characterised in that the elastic portion is at least a tongue (14) formed by incisions (12, 13) in the insertion sleeve (11).

16. Port catheter according to claim 15, characterised in that the membrane (4) is pressed against the rear end of the housing (1) by an inwardly extending collar (3) of a sleeve (2) which is preferably of metal, wherein the insertion sleeve (11) comprises an inwardly extending counterhook-shaped impressions (5) which engage with substantially complementary depressions in the outer wall of the housing (1).

17. Port catheter according to claim 16, characterised in that the tongue (14) comprises an inwardly extending offset portion (15) which engages with the counterhook-shaped impression (5) in the insertion sleeve (11) and thereby connects by mating shapes the insertion sleeve (11) to the housing (1) in the direction of rotation.

18. Port catheter according to claim 17, characterised in that the cone (7) is formed on a cap nut (6) for the attachment of a tube catheter (8).

19. Port catheter according to claim 17, characterised in that the cap nut (6) has a diameter which is equal to, or greater than, the diameter of the insertion sleeve (11).

## Revendications

1. Cathéter implanté avec un carter (1) essentiellement cylindrique dans lequel se trouve une chambre (22) qui est fermée d'un côté par une membrane (4) pouvant être percée par une aiguille d'injection et qui peut être raccordée de l'autre côté à un cathéter à tube flexible (8) par l'intermédiaire d'un moyen de liaison, avec un cône (7) à l'extrémité du carter (1) écartée de la membrane (4) pour l'implantation du cathéter par ponction, avec deux boucles d'ancrage (9, 10) qui sont en saillie depuis la paroi extérieure du carter (1) et qui peuvent être repliées et avec des moyens de maintien des boucles d'ancrage (9, 10) en position repliée,
caractérisé en ce que les boucles d'ancrage (9, 10) sont formées par un fil d'acier à ressort qui se boucle sur le carter (1) à une première position, s'étend à partir de celle-ci, depuis le carter (1) de deux côtés opposés en forme de deux boucles d'ancrage (9, 10) jusqu'à une seconde position, qui est axialement écartée de la première position, et revient au carter (1) et se bouche à nouveau sur le carter (1).

2. Cathéter implanté selon la revendication 1,
caractérisé en ce que les deux bouches d'ancrage (9, 10) peuvent être pliées dans le but du repliage, en direction d'enroulement autour du carter (1).

3. Cathéter implanté selon la revendication 1,
caractérisé en ce que les moyens de maintien des bouches d'ancrage (9, 10) sont formés par une fourchette (26) à deux dents (24, 25) qui maintiennent les extrémités des bouches d'ancrage (9, 10) repliées et en ce que la fourchette (26) présente un manche (27) ou un fil fixé sur la fourchette qui s'étend vers l'extérieur au-dessus de l'extrémité du carter (1) écartée du cône (7).

4. Cathéter implanté selon la revendication 3,
caractérisé en ce que les extrémités des dents (24, 25) sont quelque peu recourbées l'une vers l'autre.

5. Cathéter implanté selon la revendication 1,
caractérisé en ce que les moyens de maintien des boucles d'ancrage (9, 10) sont formés d'un fil (28) dont une extrémité se bouche sur les extrémités des deux bouches d'ancrage (9, 10) repliées et est maintenue par une bouche (30) qui peut être déliée au moyen de l'autre extrémité du fil (28) s'étendant vers l'extérieur au-dessus de l'extrémité du carter (1) écartée du cône (7).

6. Cathéter implanté selon la revendication 1,
caractérisé en ce que les moyens de maintien des boucles d'ancrage sont formés par un fil (31) dont une extrémité est fixée à l'une des bouches d'ancrage (10) et en ce que l'autre extrémité du fil (31) est guidée vers l'extérieur par un oeillet (33) à l'extrémité de l'autre bouche d'ancrage (9) et ensuite au-dessus de l'extrémité du carter (1) écartée du cône (7).

7. Cathéter implanté selon la revendication 1,
caractérisé en ce que les moyens de maintien des bouches d'ancrage (9, 10) sont formés par une rainure axiale (34) dans laquelle les extrémités des bouches d'ancrage (9, 10) sont encliquetées à l'état recourbé et en ce qu'un fil (35) est fixé aux bouches (9,10) en particulier dans la zone de bouchage du carter (1), qui s'étend vers l'extérieur au-dessus de l'extrémité du carter (1) écartée du cône (7) et à l'aide duquel les boucles d'ancrage (9, 10) peuvent être retirées de la rainure axiale (34).

8. Cathéter implanté selon la revendication 1,
caractérisé en ce que les moyens de maintien des bouches d'ancrage (9, 10) sont formés par une bague cylindrique (36) qui entoure les bouches d'ancrage (9, 10) repliées et est constitué en un matériau qui se dissout après l'implantation du support de cathéter.

9. Cathéter implanté selon la revendication 1,
caractérisé en ce que les moyens de maintien des bouches d'ancrage (9, 10) sont formés par un tube flexible (37, 38) qui entoure les bouches d'ancrage (9, 10) repliées, fait saillie au-dessus du carter (1) à l'extrémité écartée du cône (7) et peut en être tirée.

10. Cathéter implanté selon la revendication 9,
caractérisé en ce que le tube flexible (38) est constitué d'un matériau déchirable en direction longitudinale.

11. Cathéter implanté selon la revendication 9,
caractérisé en ce que l'extrémité du tube flexible (38) entourant les bouches d'ancrage (9, 10) repliées est repliée vers l'intérieur dans la zone des bouches d'ancrage (9, 10).

12. Cathéter implanté selon la revendication 1,
caractérisé en ce que les moyens de maintien des bouches d'ancrage (9, 10) sont formés par une douille d'insertion (11) connue en soi qui peut être glissée sur le carter (1) jusqu'aux bouches d'ancrage repliées (9, 10) et fait saillie de l'extrémité du carter (1) écartée du cône (7).

13. Cathéter implanté selon la revendication 12,
caractérisé en ce que les deux bouches d'ancrage (9, 10) forment les branches d'un V qui est ouvert en direction du cône (7).

14. Cathéter implanté selon la revendication 12 ou 13,
caractérisé en ce que la douille d'insertion (11) est en appui de frottement sur le carter (1) par une pièce élastique.

15. Cathéter implanté selon la revendication 14,
caractérisé en ce que la pièce élastique est au moins une languette (14) qui est formée par des encoches (12, 13) dans la douille d'insertion (11).

16. Cathéter implanté selon la revendication 1,
caractérisé en ce que la membrane (4) est comprimée contre l'extrémité arrière du carter (1) par une collerette (3) d'une douille (2) constituée de préférence en métal, dirigée vers l'intérieur, la douille d'insertion (11) présentant des empreintes (5) de forme barbelée dirigées vers l'intérieur, qui viennent en prise à l'arrière dans des cavités sensiblement complémentaires dans la paroi externe du carter (1).

17. Cathéter implanté selon la revendication 16,
caractérisé en ce que la languette (14) présente une coudure (15) dirigée vers l'intérieur qui s'encliquète dans la cavité (5) de forme barbelée dans la douille d'insertion (11) et relie ainsi à fermeture géométrique la douille d'insertion (11) au carter (1) en direction de rotation.

18. Cathéter implanté selon la revendication 17,
caractérisé en ce que le cône (7) est formé sur un écrou d'accouplement (6) pour fixer un cathéter en tube flexible (8).

19. Cathéter implanté selon la revendication 17,
caractérisé en ce que l'écrou d'accouplement (6) a un diamètre qui est égal ou quelque peu supérieur au diamètre de la douille d'insertion (11).
